(19) Europäisches Patentamt
European Patent Office
Office européen des brevets

(11) **EP 4 284 499 B1**

(12) **EUROPEAN PATENT SPECIFICATION**

(45) Date of publication and mention
of the grant of the patent:
**10.06.2026 Bulletin 2026/24**

(21) Application number: **22702642.4**

(22) Date of filing: **20.01.2022**

(51) International Patent Classification (IPC):
***A61N 2/02*** *(2006.01)*

(52) Cooperative Patent Classification (CPC):
**A61N 2/02**

(86) International application number:
**PCT/EP2022/051211**

(87) International publication number:
**WO 2022/161842 (04.08.2022 Gazette 2022/31)**

(54) **DEVICE FOR MAGNETIC FIELD THERAPY**

VORRICHTUNG ZUR MAGNETFELDTHERAPIE

DISPOSITIF POUR LA THÉRAPIE PAR CHAMP MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2021 DE 102021101671**

(43) Date of publication of application:
**06.12.2023 Bulletin 2023/49**

(73) Proprietor: **Gleim, Niki Reiner**
**9485 Nendeln (LI)**

(72) Inventor: **KAFKA, Wolf A.**
**82288 Kottgeisering (DE)**

(74) Representative: **Epping - Hermann - Fischer**
**Patentanwaltsgesellschaft mbH**
**Schloßschmidstraße 5**
**80639 München (DE)**

(56) References cited:
**EP-A1- 0 949 938          EP-A1- 1 044 036**
**EP-A1- 2 050 481          DE-A1- 102017 127 353**
**US-A1- 2015 119 632          US-A1- 2016 365 186**
**US-A1- 2017 058 251**

**Description**

Technical field of the invention

The invention relates to a device for magnetic field therapy as described, for example, in EP 2050481 B1.

Prior art

Since the beginning of the 1970s, devices have been known, some of which are also used routinely primarily in the field of orthopedics in clinics, medical practices and in home use for therapeutic purposes.

A generator is used to control a magnetic field generating device, whereby the generator controls the magnetic field generating device in such a way that the magnetic field consists of a large number of basic pulses or main pulses which are characteristically shaped in terms of time interval and amplitude profile and whose pulse frequency is usually between 0 and 1000 Hz. Such a main pulse can be e.g. by sinusoidal, trapezoidal, sawtooth shaped (EP 0594655 B1, EP 0729318 B1 or as in EP 0995463 B1) by an on average exponentially increasing sinusoidal modulated field intensity curve with magnetic flux densities in the range of nanoTesla to several milliTesla, in such a way that the main pulses, as for example in EP 0995463 B1, are composed of a series of temporally successive sub-pulses, which differ in their amplitudes and/or rise or fall slopes, and thus ultimately also in their individual duration.

The generation of the magnetic fields is often carried out by one or more electric coils which are also controlled independently of each other. From the multitude of devices for electromagnetic therapy, documents correspondingly oriented to the generation of time-varying field intensities can be found, for example, in EP 0995463 B1.

Today, therapeutic application is usually performed non-invasively for reasons of surgical effort and the associated risks.

The influence on the biological system is based, according to common understanding, on an interaction of the magnetic and electric field components generated by the devices, the energy components of which are still unknown. The triggered physiological and biological effects are based on the direct, by magnetic or/and according to the principle of induction (Maxwell's equations) of the indirect, by electrical force effects caused influence (energetic activation) of the physical-chemical reactivity of the ionic, atomic and molecular building blocks involved in the natural and self-preserving regulatory mechanisms, in the following referred to as molecular structures.

For example, it is described that the use of the special device EP 0995463 B1 in relation to untreated biological objects as controls leads to a number of differentiated physical-physiological processes, such as the significant activation of processes

- in the formation of energy-rich compounds, in particular adenosine triphosphate (ATP) and bis-2-3-phospoglycerate (BPG) in human erythrocytes;
- to improve the functional state of the microcirculation, in particular with regard to blood flow (especially also in diabetes-related circulatory disorders) and oxygen utilization, as well as
- in the course of protective mechanisms, especially with regard to an accelerated course of infectiously triggered leukocyte immune defense reactions supported by complex interplay of signaling and adhesion molecules;
- in protection against chemical stress factors, especially the reduction of chemically (by the teratogen cyclophosphamide) induced malformations in the ontogenesis of warm-blooded vertebrate embryos;
- in reparative processes, in particular with regard to improved healing of wounds produced by default;
- in anti-oxidative regulations, especially with regard to enzymatically and spectrophotometrically determined accelerated reduction turnover rates;
- in performance enhancement in top-level sports;
- of replication and proliferation mechanisms, in particular with regard to a significant reduction of tumor growth in thymus-free but not in comparatively examined normal mice;
- protein formation and activation, especially with regard to differential up- and down-regulation of gene-expressed protein levels;
- psychovegetative processes, in particular the reduction of (dental) anxiety by local electromagnetic stimulation of the solar plexus immediately preceding dental treatment;
- the reduction of lumbar-initiated subsequent reactions, especially the reduction of movement pain, insomnia and anxiety;
- the analgesic effect, especially with regard to the reduction of polyneuropathic pain states as a consequence of oxidative stress after chemotherapy.

Underlying problem

All devices known so far for the treatment of the human body lead to the desired accelerating effect of healing processes, but their effect can still be improved. Without taking into account whether a patient actually benefits from such a treatment, statements about it in the form of so-called surrogate parameter studies often refer only to the treatment of laboratory values such as parameters of the circulation, fat transport (LDL, HDL), lung function, bone density or other metabolic rates. This is problematic in that in order to achieve a significantly accelerated healing success, the application must be repeated frequently, often varying the field intensity ratios, which leads not only to an increased burden on the patient, but also to significantly higher treatment costs.

The relationship between the temporal intensity ratios of the applied electromagnetic fields and the respective induced biological effect is still insufficiently exploited in the device of EP 0995463 B1. According to the mentioned ideas about the effect, in particular the sub-pulses of the main pulse, which rise steeper and higher in their absolute intensity (independent of the sign) compared to the following sub-pulses, have in principle a lower potential for individual influencing of the activation energies modulating the biological reactions due to their amplitude and edge steepnesses. Simply put, the sub-pulses of a main pulse generated in immediate succession in EP 0995463 B1 are always smaller in amplitude and slope than the ones following them. Accordingly, and additionally due to the different electric field components according to Maxwell's equations, these provide only a correspondingly limited lower energetic contribution compared to the respective following sub-pulses in the sense of the desired activation of regulatory mechanisms spread as widely as possible.

With reference to the envelope curves described in EP 0995463 B1 as a connection of the upper and lower extreme values of the sub-amplitudes, it is added that the envelope curve closer to the coordinate axis increasingly moves away from it within the pulse train: Measured in terms of amplitude, successive sub-pulses would therefore have a lower activation potential compared to those in which the envelope curve described moves less "away" from the coordinate axis. - Accordingly, in the sense of the desired broad activation, the forms of the main and sub-pulses described in EP 0995463 B1 are still insufficiently graded in terms of duration and amplitude and amplitude sequence.

In a further development described in EP 2050481 B1, the intensity progression over time was adjusted so that the pulses are more finely adapted to the requirements of the therapy. The optimal form and sequence of the sub-pulses varies greatly from individual to individual. It depends on the type of tissue impacted by the field, the desired healing outcome, and the individual. The high proportion of rising and falling flank sections, caused by the large number of sub-impulses, is crucial in stimulating the exchange processes in the body's tissues. Thus, a significant stimulation of the body on a molecular level can be achieved. Higher physical levels, however, are not addressed or only insignificantly.

Objective and solution

An object to be achieved is to specify an improved concept for magnetic field therapy with a driving signal which, in addition to a broader spectral composition, is characterized by targeted effects on superordinate physical levels.

This object is achieved by the independent claim, wherein advantageous further embodiments are specified in the dependent claims.

The improved concept is based on the idea of, in addition to the signal form described in EP 2050481 B1, which stimulates the body at the molecular level, also influencing regulation at physiologically higher levels in the actuation signal with sequences of electrical signal events suitably timed in terms of duration and intensity, for example the coordination or synchronization of networked physiological reaction sequences. In particular, this is achieved by superimposing a rhythmic signal which comprises a sequence of signal events organized in at least one regular stress pattern. It has been shown that it is particularly the combination with the signal acting at the molecular level that leads to the fact that, for example, the regulatory mechanisms at a physiologically higher level can be better achieved by the basic activation at the molecular level. Thus, the regular stress patterns can have an increased influence on, for example, physical regulatory mechanisms.

This applies not least to a treatment (entrainment) oriented to the performance adaptation and sensitivities of recurring bodily functions.

The use as load-dependent entrainment for the regulative synchronization of endogenous, also bio-rhythmic processes is particularly promising here.

This includes natural and biorhythmic body functions that fluctuate in time ranges from milliseconds to years, such as anxiety, stress, performance, balance, insomnia, respiratory and heart rate, as well as the following the mechanisms of cell division and renewal regulated by internal timers, endocrine release and activation of signaling substances and proteins, vasomotion, digestion, menstruation, as well as the aperiodic regulatory mechanisms induced by light, color, weather, temperature, gravity, sound, physical and mental performance demands, including the synchronization of molecular oscillators of intracellular and intercellular networks , including their timer functions.

Activation probability may increase with increases in both impulse intensity and density of impulse sequences.

Accordingly, relaxing effects, e.g. after high physical performance demands, can be achieved by exposure to stimulation with temporally essentially decreasing pulse density and/or intensity, whereas performance-enhancing effects can be achieved with temporally increasing density and/or intensity values. A treatment aiming at inner peace and balance can be achieved, for example, by stimulations with largely constant density and/or intensity values.

According to the improved concept, for example, a device for influencing biological processes in a living tissue, in particular a human body, for applying a pulsating magnetic field to at least a part of the tissue comprises a field generating device for generating the pulsating magnetic field and a pulse generator for driving the field generating device. For this purpose, the pulse generator is adapted to output a signal sequence which is formed from a superposition of at least one sequence of main pulses whose pulse repetition rate is in each case between 0.1 and 1000 Hz, for example between 1 and 3 Hz as the lower limit and 200 to 300 Hz as the upper limit, and a rhythmic signal. The rhythmic signal comprises a sequence of signal events organized in at least one regular stress pattern.

Therein an amplitude waveform of a main pulse comprises the following function y(x):

$$y(x) = k1 + k2 \cdot e^{\sin(x^{k3}) + \sin((x \cdot k4)^{k5}) + x \cdot k6}.$$

Therein mean:

x = computational substitute for time t during a main pulse;
k1 = offset value;
k2 = amplitude factor, k2≠0;
k3 = exponent of x, k3≠0;
k4 = multiplication factor of x, k4≠0;
k5 = exponent of (x*k4), k5≠0;
k6 = multiplication factor of x;
wherein k1 - k6 are parameters which are freely selectable within certain limits to give different shapes to the amplitude waveform, wherein each main pulse is modulated with sub-pulses (11) by respective selection of the parameters.

The function describes an exponential modulation course essentially defined by the parameters k1, k2, k3, k4, k5 and k6, wherein this modulation course is governed by two SIN functions determined by the parameters k3, k4 and k5. Since a SIN function can only assume values between +1 and -1, the sum of the two SIN functions determining the e-function lies between -2 and +2. The e-function can therefore only assume values between $e^{-2} = 0.135$ and $e^2 = 7.39$, if one disregards the further term +x*k6, which then defines a further increase. The SIN functions themselves with the associated parameters are chosen in such a way that a fine gradation of the successive amplitude values in the sub-pulses defined with them is achieved with a high range of variation.

The selectable parameters k1 to k6 have the following meaning and effect on the signal sequence, in particular on the main pulses forming the signal sequence:

- k1 is a presettable basic amplitude value with which a basic signal value or bias value can be defined on which the main pulses "touch down" (zero line symmetry or zero line asymmetry). This basic value does not have to correspond to a fixed amplitude value, but can vary individually in the time sequence for each main pulse.
- k2 (k2≠0) is a multiplication factor for the e-function; the larger k2 is selected, the larger is the maximum achievable modulation amplitude value of a main pulse, which is added to the basic amplitude value. k1 and k2 can be limited - depending on k6 - in such a way that the permissible field strengths, which differ in individual countries, are not exceeded.
- k3 determines as a kind of time-dependent scaling factor together with k4 and k5 the time-dependent course (slope and amplitude) of the modulated signal or magnetization value of the individual sub-pulses. The larger the parameter k3 is chosen, the larger the "ripple" of the envelopes connecting the extreme values of the sub-pulses.
- Together with k3 and k5, k4 also determines the number and slope of the individual sub-pulses, but in particular, as a kind of density factor, the time sequence of the individual sub-pulses , (k4≠0); the larger the parameter k4 is selected, the more densely the individual sub-pulses are embedded in the individual "waves" of the envelopes of each main pulse.
- k5 is, similar to k3, a kind of temporal scaling and dimensioning factor, by which In conjunction with the parameters k3 and k4 the temporal modulation course (slope, amplitude, and density in the sequence within the "waves" described above) of the signal or magnetization value of the individual sub-pulses within a main pulse can be adjusted.
- k6 is a normalization and dimensioning factor for setting an average amplitude value which depends on k1 and k2 and can be fixed for the sequence of the individual main pulses. It specifies with which slope the amplitude of the main pulse increases or decreases during the "active" pulse duration. k6 thus also describes the average amplitude increase of the sub-pulses embedded in the main pulse. For example, values k6 > 0 lead to increasing amplitude values of the sub-pulses on average, and values k6 < 0 lead to decreasing amplitude values on average - especially illustrated by the envelopes connecting the extreme values of the sub-pulses. This has a particular effect on the envelope facing away from the abscissa axis. With k6 = 0, the amplitude values of the sub-pulses remain constant on average and the envelopes run parallel to the x-axis. The parameter k6 can be given as k6≠0.

The parameters of the function y(x) can be chosen, for example, with the properties k3 to k5 being integer and k1, k2 and k6 selected in decimal increments from the following ranges of values:

- 

$$6 < k1, k2, k6 < 6 \text{ (particularly in steps of } 0.1);$$

- 

$$6 < k3, k5 < 6 \text{ (integer)};$$

- 

$$10 < k4 < 10.$$

For example, the computational substitute x depends linearly on the time t.

For example, the computational substitute x for time t is defined as

$$x = x1 + \frac{x2 - x1}{T_i} \cdot (t - t0_i),$$

with

x1 = initial value for x;
x2 = final value for x;
Ti = pulse length of a main pulse; and
t0i = start time of the main pulse.

Thus, a linear progression for the value x between the initial value x1 and the final value x2 ultimately results, independent of the pulse length of the main pulse concerned. In other words, the course of x between x1 and x2 is stretched or compressed by a changing pulse length.

Signal events are, for example, uniquely identifiable signal forms that usually extend over a defined period of time. Furthermore, the signal events can comprise a certain overall intensity, wherein a signal event itself can assume a time-varying intensity course. The organization of the signal events in a regular stress pattern makes it clear that this stress pattern must occur repeatedly and differs in particular from, for example, individual signal pulses.

For example, the stress pattern is formed by a temporally defined occurrence of the signal events. This enables, for example, the formation of a rhythm in the rhythmic signal by the stress pattern.

For example, the signal events of the rhythmic signal may all comprise the same or a similar signal pattern. However, the rhythmic signal may also be formed by different or dissimilar signal events.

By similar it is understood, for example, that there is no complete identity between two signal events, but a strong connection, for example by similar time course, similar spectral composition, similar type of generation.

For example, at least one group of similar signal events emerges periodically from the sequence of signal events. Thus, so to speak, a rhythm and/or a beat of the rhythmic signal can be formed.

For example, the stress pattern defines a clock measure, such as by selected signal events from the sequence of signal events. For example, the clock measure specifies the time intervals at which the stress pattern is basically repeated. The clock measure can be constant, so that the stress pattern is regularly repeated at constant time intervals. This makes it possible, for example, to achieve a treatment aimed at inner calm and balance in the sense of entrainment. However, in various embodiments, the tempo of the beat can also be varied, for example with increasing tempo or decreasing tempo. However, this does not change the basic regularity of the stress pattern in the rhythmic signal. With a falling tempo of the beat measure, for example, relaxing effects can be achieved, while with a rising tempo performance-enhancing effects can be achieved.

In order to achieve an effective influence on the physiologically superordinate levels, it has been found that at least a sufficient number of repetitions of the stress pattern in the rhythmic signal is required. For example, the stress pattern in the rhythmic signal is repeated at least 10 times per minute, in particular at least 50 times per minute. In practical applications, however, significantly higher numbers of repetitions have also been found to be useful.

This is also reflected, for example, in a preferred length of the resulting signal sequence, which is, for example, between 45 seconds and 25 minutes, or even between 90 seconds and 20 minutes. An optimal treatment duration, i.e., length of the signal sequence is about 8 to 17 minutes.

The signal events comprise, for example, a frequency range such that, when the rhythmic signal is reproduced via an electroacoustic transducer, an auditory sound and/or an infrasound results. Indeed, it has been shown that the body reacts in particular to such a frequency range for stimulating or influencing the physiologically higher levels. However, these effects do not result from the acoustic stimulus, but are given by the pulsating magnetic field, which results inter alia from the rhythmic signal.

For example, the signal events are formed by at least one of the following: A breathing sound, a blood flow sound, a sound of a heartbeat, a sound of a percussion instrument, a sound of a stringed instrument, a sound of a wind instrument, or more generally a sound produced by any technology or natural event, e.g., fireworks, mood sounds, water waves, ocean noise, fireplace sounds. If signal events are formed by sounds of the same kind, such as the sound of a heartbeat, this also represents a similarity of these signal events.

In a further development, the signal sequence is formed by a superposition of a first sequence of main pulses, at least a second sequence of main pulses and the rhythmic signal. The amplitude course of the main pulses comprises the function $y(x)$ defined above. Thereby, each main pulse comprises a pulse length. The pulse length and the pulse repetition rate of the main pulses of the second sequence of main pulses are different from the pulse length and the pulse repetition rate of the main pulses of the first sequence of main pulses. Further, the pulse length of the main pulses of the second sequence of main pulses changes monotonically in a time-dependent course. For example, the pulse length decreases monotonically or increases monotonically. The pulse repetition rate of the main pulses of the second sequence of main pulses also changes monotonically over time, in the opposite direction to the change in pulse length. For example, the pulse repetition rate monotonically increases or monotonically decreases. Thus, if the pulse length increases, the pulse repetition rate decreases and vice versa.

The improved concept ensures, for example, a broader physiological range of effects by addressing a broad band of electromagnetically activatable molecular structures, which further enhances the positive effect of the rhythmic signal. The improved stimulation possibilities of molecular structures thus ensure an extended physiological range of action, in particular through interaction at higher levels on the basis of the rhythmic signal.

The improved concept is thus oriented to the broadest possible energetic support of the complexly interconnected molecular regulatory processes that determine the normal course of life, which strengthen the stimulation of the bodily regulatory mechanisms on a higher level by the rhythmic signal. Deviating from the usual symptom-oriented treatment methods, it accordingly pursues a holistic and thus preventive therapy concept, as well as a therapy concept oriented towards regeneration, maintenance and well-being.

The improved concept is based on the idea of continuously varying the sequence of different electro-magnetic stimulation signals during treatment, in particular by superposition of at least two similar stimulation signals, especially sequences of pulses with different parameters regarding pulse length and pulse repetition rate. This is done in particular by varying pulse length and pulse repetition rate in at least one of the sequences to be superimposed. The variation of the sequence of such signal sequences generated by superposition, interrupted by possible pauses, can also be used. Advantageously, compared to conventional methods, different tissue parts can be exposed to temporally varying signal patterns in the course of a treatment in this way (due to intensity or range). Depending on the type of superposition and signal sequence, in particular also periodically and/or rhythmically repeating physiological and bio-rhythmic processes such as respiratory and heart rates, sleep rhythms, etc. could thus be influenced or supported.

Accordingly, both the first sequence and the at least one second sequence each consist of a certain number of main pulses, each following said function $y(x)$. However, the parameters $k1$ to $k6$ of the first sequence may differ from corresponding parameters of the second sequence, as well as between different main pulses within a sequence. The different pulse length results in particular from the used approach, how the arithmetic substitute $x$ for the time $t$ results.

A respective main pulse is composed, for example, of an amplitude sequence of sub-pulses with different edge steepnesses which remains constant on average or increases or decreases on average in the manner of a power function. Characterized by connecting lines of the extrema, also envelopes, of the individual sub-pulses, the main pulses can assume a pulse-shaped course depending on the selected conditions.

As already explained above, a linear progression for the value $x$ between an initial value $x1$ and a final value $x2$ ultimately results regardless of the pulse length of the main pulse concerned. In other words, the course of $x$ between $x1$ and $x2$ is stretched or compressed by a changing pulse length.

In this case, the pulse length $Ti$ of the main pulses of the second sequence of main pulses can change in a time-dependent course, for example, linearly, logarithmically or exponentially.

The pulse generator is used to drive the field generation device, wherein the pulse generator drives the field generation device via suitable current or voltage sequences in such a way that the pulsating magnetic field resulting from the signal sequence is composed in such a way that the pulsating magnetic field resulting from the signal sequence has as large a spectral width as possible.

In the case of voltage control, it must be ensured that the inductance of the coil means that the applied voltage signal does not result in a magnetic field signal that corresponds to the mathematical curve of the voltage signal, because the inductance of the coil reproduces the signal curve at higher frequencies with lower amplitudes. This can be counteracted, for example, by transferring the desired time function of the magnetic field into the frequency domain via a Fourier transformation, where the high frequencies are raised accordingly in order to then obtain the appropriate time signal for the voltage waveform.

Another possibility is to use a voltage-controlled current amplifier, which applies current signals to the coil with a waveform that corresponds to the desired magnetic field signal. The superposition of the first and second sequences to form the signal sequence also results in different spectral characteristics of the individual sequences due to the different pulse lengths and pulse repetition rates, which ultimately leads to a superposition of spectral characteristics in the signal sequence output by the pulse generator. Furthermore, the monotonic change in pulse length also results in a changing spectral property over time, so that interactions of different frequency components can be achieved in the pulsating magnetic field and thus in the impacted tissue.

Because of the changing pulse lengths and the resulting frequency shifts within the main pulses, changing frequency deviations between the signal of the first sequence and the signal of the second sequence thus follow. This, in turn, can lead to beat-like conditions, which result in further stimulation of the impacted tissue.

Due to the differential effects on tumor growth and gene expression, the effects cannot be explained with an improved microcirculation, but confirm and imply the assumption stated in the introduction that the electromagnetically induced biological effects are based on the activation of causally different molecular mechanisms. It is assumed that the different processes consequently require different amounts of energy for their activation. The distribution of the amplitudes, the embodiment of the slopes and the superposition of the sub-pulses are therefore of decisive importance, since with these parameters the intensity distribution over time is characterized. As a kind of electromagnetic agent, the temporal field intensity distributions are therefore of similar importance to the structure-activity relationship of active pharmaceutical ingredients.

Instead of chemical formulas, it could be quantified according to well-known rules of school mathematics as an additive superposition of sine and cosine components suitably superimposed with respect to frequency and amplitude, e.g. as a characteristic amplitude-frequency (Fourier) spectrum. The broader the amplitude-frequency spectrum of the applied fields, the broader the activation possibilities and thus ultimately the more efficient and broadly spread the expected biological effect would be.

However, even if a device with a broadband field application already contains many of the intensity time courses used in other devices, it should not be assumed that this also covers their effects. In addition - and thus similar to medication with multifunctional agents - synergistic effects (resulting from their interaction) must also be taken into account here. In this respect, the sequence of different electro-magnetic signal sequences of the type described - also interrupted by pauses - can also play a role.

In various embodiments, the main pulses of the first sequence of main pulses follow each other directly in the time-dependent course, in particular without a pause. Similarly, the main pulses of the second sequence of main pulses also follow one another immediately in the time-dependent course, in particular without a pause. This can result, for example, in the start times of the individual main pulses in the first and second sequences moving further apart with each main pulse, and thus also in the spectral components within the main pulses overlapping at different times in order to achieve the broadest possible excitation spectrum.

In alternative embodiments, the main pulses of the first and/or second sequence may also follow each other with pauses. These may comprise a constant length, or they may comprise a variable length corresponding, for example, to the change in pulse length.

In various embodiments, the pulse length of the main pulses of the first sequence of main pulses may remain constant, while the pulse length of the main pulses of the second sequence changes.

However, in various embodiments, it is also possible for the pulse length of the main pulses of the first sequence of main pulses to change monotonically over time, for example to increase monotonically. In this case, the pulse repetition rate of the main pulses of the first sequence of main pulses changes in the time-dependent course in the opposite direction to the change in the pulse length of these main pulses, for example increases monotonically or decreases monotonically. The variation in pulse length and pulse repetition rate in the first sequence of main pulses results in a further change in spectral characteristics over time, so that in turn a further broadening of the frequency spectrum acting on the tissue can be expected.

In various embodiments, the pulse generator is adapted to output the signal sequence as a first signal sequence and at least one further signal sequence in temporal succession, the at least one further signal sequence being different from the first signal sequence. The first and the at least one further signal sequence are thereby composed according to the principle described above, namely by superimposing at least one sequence of main pulses and the rhythmic signal. For example, the signal sequences differ with respect to the pulse length of the main pulses and/or one or more of the parameters k1 to k6.

For example, the pulse generator is adapted to output the at least one further signal sequence after a defined pause time after the output of the first signal sequence.

The present device leads to a faster and broader excitation of the molecular interactions and metabolic processes involved in the regulatory mechanisms. With regard to the fundamental significance of these regulatory mechanisms, which are crucial for the course of life, such field excitation can thus achieve more beneficial effects in a wide variety of medical applications. For example, in relation to the improvement of the functional state of the microcirculation and the associated increase in $O_2$ utilization, this leads to a further increase in the production of the energy carrier ATP, which energetically supports the processes of transcription, translation, formation and modulation of the activity state of proteins, and - as a further consequence - also to an accelerated setting of proteomics, i.e. a more efficient provision of the proteins modulating these regulations.

In particular, this can also lead to further improved support of the extremely complex sequence of immune defense reactions characterized by signaling molecules and various forms of adhesion molecules.

In conjunction with increased synthesis performance, increased $O_2$ utilization leads, inter alia, on the one hand to increased connective tissue and cartilage formation and additional vascularization and thus also enhances the formation of chondrocytes, which is strongly dependent on $O_2$ diffusion in the cartilage. Due to a shape-retaining and regeneration-promoting effect of this bone formation promoted by electromagnetic pulses, the organism is thus able to repair the necessary structures and injury- and disease-related disturbances in bone formation in an accelerated manner with a minimum of material and energy.

On the other hand, the bioelectrical effect of the induced tensions, in connection with an activation of the proton pumps supporting the ATP formation and further favored by the $O_2$ utilization, can lead to a mineralization of the connective tissue via an increased ion exchange. Because of the build-up and degradation processes of cartilage, which are closely coupled to bone metabolism, the device may also influence the calcium influx and efflux kinetics of chondrocytes, which are co-decisive for the consolidation of bone fragments.

The membranes of the membrane systems, which are important for intracellular and intercellular signaling and substance transfer, are affected either directly or by the potentials formed in the collagen, or also via a change in the microenvironment of the cell. This mechanism, possibly also as above in conjunction with electromagnetic support of proton transport mechanisms, is presumably based on electrochemical transmission that modifies cell activity by shifting the ionic atmosphere in the extracellular and thus also in the intracellular space. The capacitive charging of the cell membrane by the electrical component of pulsating electromagnetic fields represents a crucial factor in this process. Caused by the structural and charge shift in the membrane, especially in the area of the pores, there is the possibility of a permeability change with a resulting influence on passive ion transport and diffusion processes.

Due to the close coupling between surface reaction and transmembrane transport, other membrane-bound proteins, such as the Na-K pump, appear to be important receiver structures for the induced energy in addition to the proton pump. In this context, increased Na-K adenosine triphosphatase activity can cause increased sodium supply by the responsible ion pump. In this context, only excitation with an optimal amplitude profile of the main pulses according to the invention leads to excitation of the active transport complexes via presumably an increase in the surface concentration of the corresponding ions.

In its positive effect on the complexly interconnected, multilayered regulatory processes in the course of life, the list of advantageously supported individual mechanisms could be further extended in this way. Apart from the aforementioned general positive effect, also on competitive sports, the device can thus contribute quite generally to the optimized support of general natural protective, healing and maintenance processes and well-being.

In conjunction with the reduction of the occurrence of general chronic disorders, this invention could ultimately also further limit the development of general illness costs via its influencing of vital processes of substance and energy turnover and thus also the reduction of medications linked thereto.

What is particularly advantageous about the proposed device is that it can also lead to a stimulation of metabolic processes throughout the patient's body in the case of local treatment.

Good results can also be achieved if, during a treatment period, the parameters of the amplitude function y are not kept constant, but are varied according to a pattern tailored to the patient. For this purpose, groups of parameters are defined which are then applied successively in time periods to be selected.

With special, previously described settings, in particular of the rhythmic signals, the treatments thus allow promising successes to be expected in the broad region of the levels of physical, physiological and also psychological sensitivities that are superimposed on the molecular mechanisms.

Appropriately adjusted devices, e.g. incorporated into clothing, could positively contribute to the willingness and ability to perform by coordinating the rhythmic signal with, for example, the walking rhythm or/and the heart or/and breathing rate. This also applies to the basic needs for rest, sleep and breaks, as well as to stress, depression, burnout and anxiety caused specifically by stress.

The improved concept is explained in more detail below by means of exemplary embodiments with reference to the drawings. Here, elements of the same kind or elements of the same functions are designated with the same reference signs. Therefore, a repeated explanation of individual elements may be omitted.

In the drawings:

Figure 1    shows a schematic representation of an embodiment of a device for influencing biological processes,
Figure 2    shows a principled time-dependent course of a main pulse,
Figure 3    shows a time-dependent course of a sequence of main pulses and a rhythmic signal before superposition and a signal sequence after superposition,
Figure 4    shows a principled time-dependent course of main pulses from a first and a second sequence before superposition,
Figure 5    shows a principled time-dependent course of a first and a second sequence before the superposition as well as of a signal sequence after the superposition,
Figure 6    shows a principled time-dependent course of a sequence of superimposed main pulses according to Figure 5 and a rhythmic signal before superposition and a signal sequence after superposition, and
Figure 7    shows a principled time-dependent course of a sequence of superimposed signal sequences.

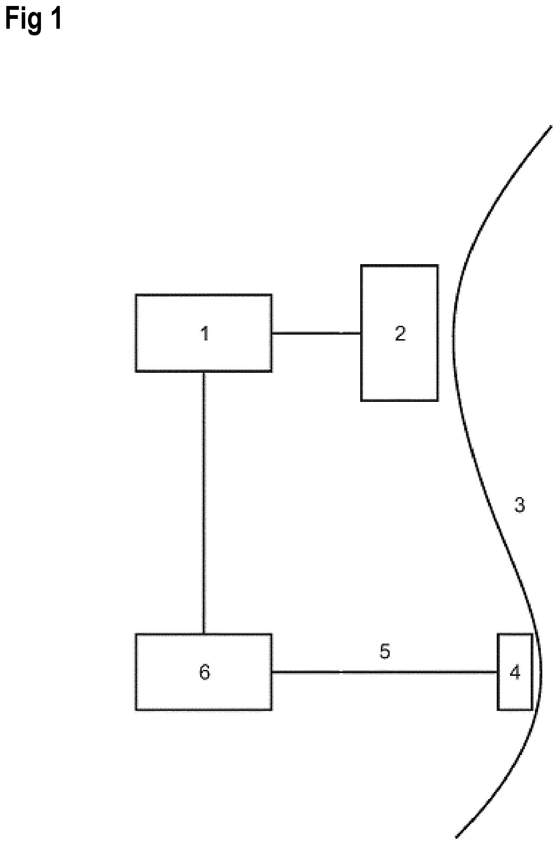

In detail, Figure 1 shows a device according to the improved concept, comprising at least one pulse generator 1, which generates a pulsating magnetic field in a coil 2, which takes effect in the living tissue 3, in particular in the body of a patient to be treated. In order to adjust, in particular optimize, the pulse parameters of the pulsating magnetic field in the pulse generator 1, an optional sensor 4 can detect certain body parameters. Such body parameters include, for example, temperature, blood pressure, pulse rate, skin resistance or blood oxygen content. The sensed parameter is fed via a feedback line 5 to a control unit 6, which evaluates the parameter and controls the pulse generator 1 accordingly. For improved optimization, it is possible to record and evaluate several body parameters simultaneously for optimization of the pulsating magnetic field. Depending on these effects, the control unit 6 can also automatically determine the optimal values for the parameters k1 to k6 in each case.

In addition, a sensor for detecting the frequency dependence of the effect of the field generating device 2 transmitted to the body can optionally be provided. From the differences, in particular in the spectral composition between the field energy generated by the field generation device and the spectrum detected by the sensor, the control unit determines the portion transmitted to the treated body. Depending on this effect, the control unit 6 itself determines the optimum values for the parameters k1 to k6. In such field generating devices 2, the field strengths can additionally be varied within the geometry of the field generating device 2.

With the device according to the improved concept, a pulsating magnetic field is generated in such a way that a signal sequence is delivered from the pulse generator 1 to the field generating device, which is formed from a superposition of at least one sequence of main pulses and a rhythmic signal.

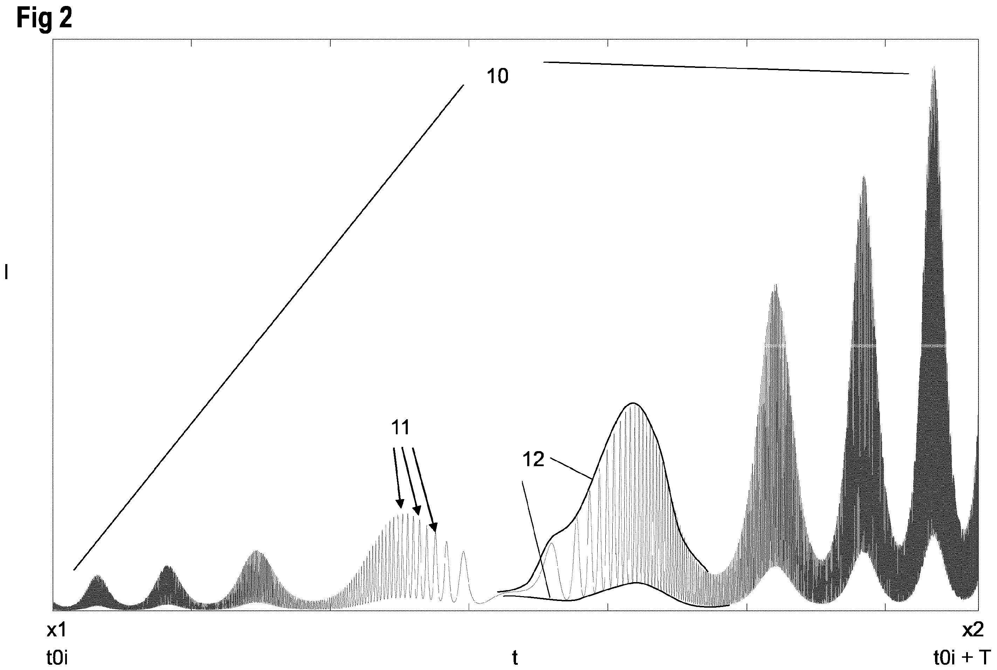

Figure 2 shows a principal time-dependent course of the intensity I of such a main pulse 10, which is modulated with sub-pulses 11. The main pulse 10 follows the already described function y(x)

$$y(x) = k1 + k2 \cdot e^{\sin(x^{k3}) + \sin((x \cdot k4)^{k5}) + x \cdot k6}.$$

With the parameters k1 to k6 already described. The main pulse 10 starts at time t0i and ends at time t0i+T, where T is a value for the pulse length. Since the function y(x) is based on the computational substitute x for the time t, this results in a course of the function between x1 and x2. The choice of the values of x1 and x2 is in principle arbitrary, but lies for example in the range from -10 to 0 for x1 and 0 to 10 for x2, thus for example x1 = -5 and x2 = +5. The curve of the intensity I of the main pulse 10 in Figure 2 shows that the density and thus also the steepness of the sub-pulses 11 following one another in the main pulse 10 and thus also the "ripple" (pulsations of the envelope curve 12 connecting their extremes) increases constantly depending on the respective x-values or t-values.

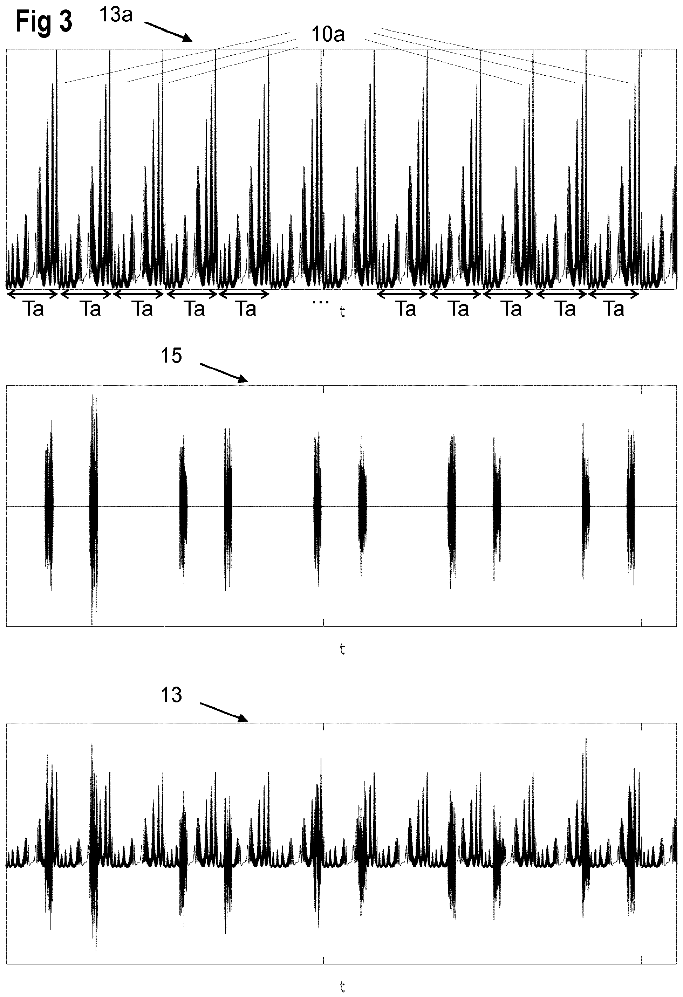

Figure 3 shows in the upper graph a sequence 13a of main pulses 10a, for example each corresponding to a main pulse shown in Figure 2. The main pulses 10a each comprise a pulse length Ta, which is kept constant in this exemplary embodiment. However, in variations, the pulse length Ta of the individual main pulses can also be variable, for example increasing with time or decreasing with time.

The middle graph of Figure 3 shows an example of a rhythmic signal 15, which here corresponds, for example, to the sound of a heartbeat. The signal events of the rhythmic signal 15 are formed here by the respective single beats of five successive double heartbeats, which are repeated in the regular stress pattern. The stress pattern is formed in particular by the respective double beats and by the repeating double beats. **In** the lower graph of Figure 3, the superposition of sequence 13a with rhythmic signal 15 is shown.

It should be noted that in all three graphs of Figure 3 the time scale is chosen identically. Furthermore, the graphs each show only a temporal section of the corresponding signals, since a complete representation of the signal, which is composed, for example, of a plurality of main pulses and a plurality of double beats of the heartbeat, would no longer ensure a comprehensible representation. The displayed section corresponds to about 5 seconds, while the complete signal can comprise a length of about 45 seconds up to 25 minutes or longer.

There is no identity of the individual heartbeats in the example shown, but there is a similarity of the corresponding signal events, which together form the stress pattern of the rhythmic signal. The individual double beats also define a beat measure, the beats per minute, so to speak, on which the rhythmic signal is based. While in the variant shown the beat measure is essentially constant, in alternative embodiments this can also be increased in tempo or decreased in time-dependent course. The different variants can thus be used to achieve different entrainment objectives, such as a treatment aimed at inner calm and balance with an essentially constant beat, relaxing effects with a decreasing beat, or stimulating effects with an increase in the beat's tempo over time.

If the rhythmic signal were reproduced solely via an electroacoustic transducer, such as a loudspeaker, this would result in an auditory sound due to the selected frequency range of the signal events. However, in the present application, no acoustical reproduction is performed, but the signal is used together with the sequence of main pulses to excite an electromagnetic field.

Nonetheless, other signal events may be selected for the rhythmic signal that would also produce an audible sound and/or also an infrasound if played through an electroacoustic transducer. Examples of alternative signal events include, in addition to the sound of a heartbeat, a breathing sound, a blood flow sound, or sounds produced by musical instruments. As examples, the sound of a percussion instrument, a stringed instrument, a wind instrument may be mentioned without claiming to be exhaustive. In particular, signal events of different nature or origin can be combined for the rhythmic signal to form the regular stress pattern.

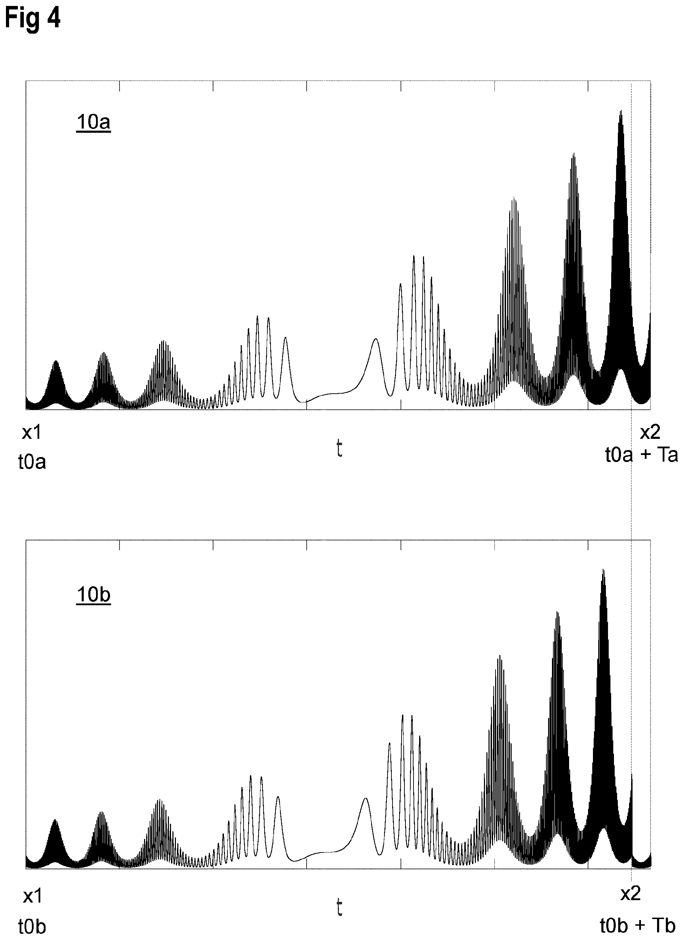

Figure 4 shows a principled time-dependent course of a further development of the improved concept, in which a first and a second sequence of main pulses 10a, 10b with different pulse lengths Ta and Tb are superimposed, so to speak as a basis for a further superimposition with the rhythmic signal. In particular, the pulse length Tb is shorter than the pulse length Ta in this representation. Nonetheless, both main pulses start at the value x1 and end at the value x2, by corresponding linear mapping of the time t to the computational substitute x.

In the illustration of Figure 4, the parameters k1 to k6 are chosen to be the same for the main pulse 10a and the main pulse 10b. This results in the present example in a temporal compression of the main pulse 10b in comparison to the main pulse 10a, which also results in a changed frequency behavior, in particular in increased spectral components.

The representation in Figure 4 corresponds, for example, to the respective first main pulse of the first sequence of main pulses 10a and second sequence of main pulses 10b. Thus, for example, the starting times of the two main pulses 10a, 10b coincide.

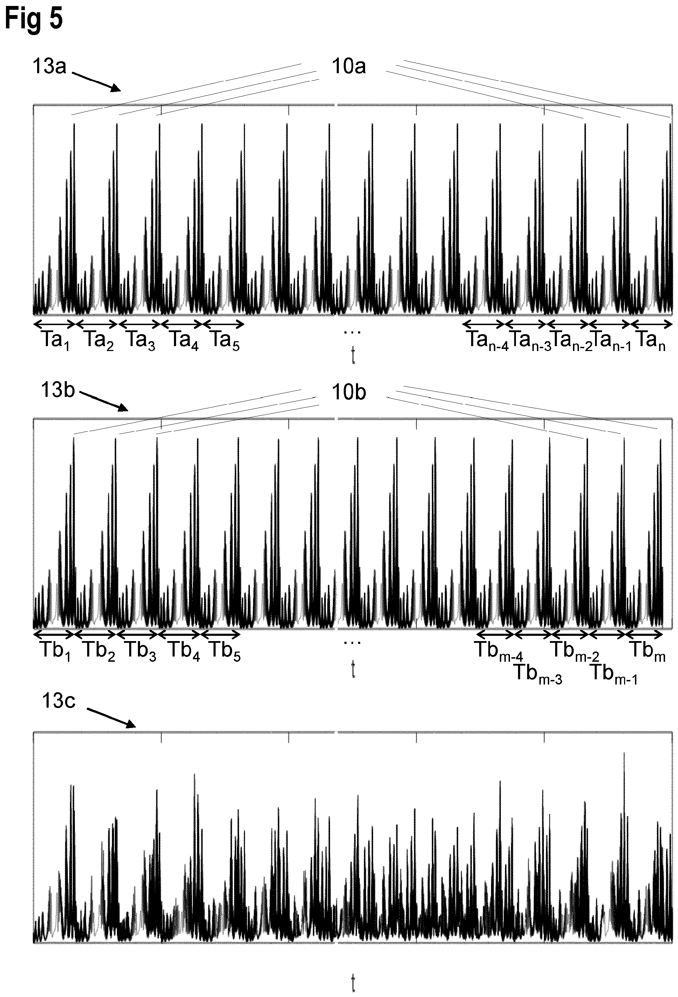

In Figure 5, the upper graph shows a first sequence 13a of main pulses 10a, each comprising a pulse length $Ta_i$ with i = 1 ... n. Similarly, a second sequence 13b of main pulses 10b is shown in the middle graph, each comprising pulse lengths $Tb_i$ with i = 1 ... m.

In the example shown, the pulse lengths $Ta_i$ of the first sequence 13a are constant, while the pulse lengths $Tb_i$ of the second sequence 13b of main pulses 10b decrease linearly. However, in addition to a linear change, a logarithmic or exponential progression could also be selected. Likewise, it is possible that the pulse length $Ta_i$ also changes accordingly and, in particular, is non-continuous, wherein, according to the improved concept, the pulse lengths of corresponding main pulses in the first and second sequences 13a, 13b are nevertheless different.

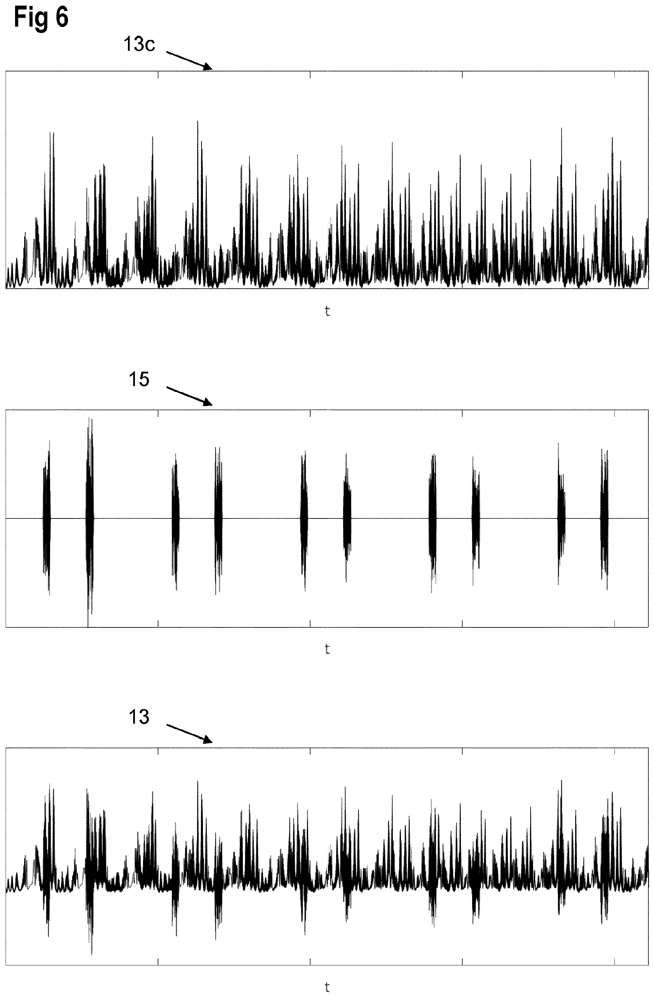

The lower graph of Figure 5 shows a sequence 13c resulting from a superposition of the first and second sequences 13a, 13b. This sequence 13c is, for example, the basis for a superposition with the rhythmic signal 15, as described in connection with Figure 3 and further explained below at Figure 6.

For reasons of clarity, a number of 15 and 16 main pulses, respectively, has been selected for the number of main pulses of the first and second sequences 13a, 13b, for example, wherein this number may be considerably higher in practical applications in order to be able to realize corresponding treatment durations. However, this does not exclude the generality of the explanations, but merely serves to make visible the principle of the constantly changing temporal shift and shortening of the main pulses 10b of the second sequence 13b in comparison with the first sequence 13a. In the sequence 13c, it can be clearly seen that the superposition results in different spectral intensities depending on time, even if a time representation is selected here.

If the individual main pulses within the first or second sequence immediately follow one another, the pulse repetition rate results implicitly from the respective pulse length, wherein the pulse repetition rate thereby changes indirectly proportional to the pulse length. For example:

Pulse repetition rate = 1/ pulse length

Figure 6 shows in the upper graph, following Figure 3, a section of the sequence 13c as it resulted approximately in the lower graph of Figure 5. The middle graph of Figure 6 again shows a section of the rhythmic signal 15 with five double heartbeats. The bottom graph of Figure 6 shows the superposition of sequence 13c with rhythmic signal 15 as the resulting signal sequence 13.

The rhythmic signal 15 in the exemplarily chosen form again serves only to visualize the improved concept. For possible alternatives in the selection of the rhythmic signal 15, reference is made to the corresponding explanations at Figure 3.

Due to the improved effect of the magnetic field signal based on the superposition of main pulses of varying pulse length and pulse repetition rate, a further improved activation of molecular effects is achieved in the body, which serves as a basis for a successful influence on a physiologically higher level, for example physical regulatory mechanisms. Thus, both the improved effects at the molecular level and an improvement in the influence on the physical regulatory mechanisms are achieved.

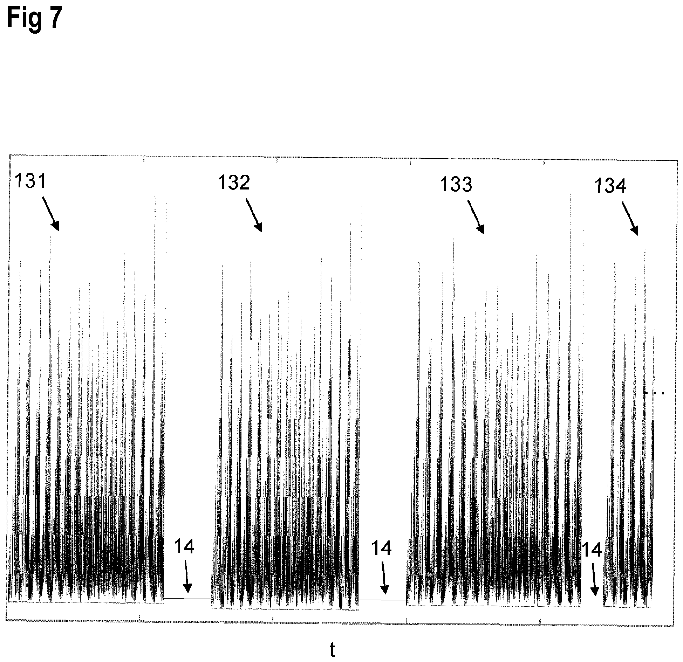

Figure 7 shows the principled time-dependent course of a sequence of superimposed signal sequences of the type described in connection with Figures 4 and 5. This sequence may also include a rhythmic signal 15. For example, the pulse generator 1 is configured to output several signal sequences 131, 132, 133, 134 and so on in succession, wherein these may differ, for example, in the pulse length of the main pulses of the first sequence or the second sequence of main pulses. It is also possible to vary the parameters k1 to k6 between the individual signal sequences 131, 132, 133, 134 and so on, and this permits individual design of the magnetic field to be generated with regard to an optimum effect on the biological tissue applied. Individual defined pause times 14 can be inserted between the signal sequences, which can be designed to be constant or also variable between the different sequences. In principle, the pause time can also be selected to be zero. In the case of superposition with the rhythmic signal 15, the latter can be superimposed continuously or alternatively suspended or interrupted in the pause times 14.

The superposition of a rhythmic signal on sequences of main pulses presented in the present disclosure is also applicable to any other forms of electromagnetically pulsed exposures, in particular also with such main pulses which do not directly fall under the above-mentioned formula y(x).

## Claims

1.    A device for influencing biological processes in a living tissue, in particular a human body, for applying a pulsating magnetic field to at least a part of the tissue, with a field generating device (2) for generating the pulsating magnetic field and a pulse generator (1) for driving the field generating device (2) with a signal sequence (13), the pulse generator (1) being adapted to output the signal sequence (13), which is formed from a superposition of at least one sequence (13a) of main pulses (10a), the pulse repetition rate of which is between 0.1 and 1000 Hz, in particular is between 3 and 300 Hz, and a rhythmic signal, wherein

- the rhythmic signal comprises a sequence of signal events organized in at least one regular stress pattern; and
- an amplitude waveform of a main pulse comprises the following function y(x):

$$y(x) = k1 + k2 \cdot e^{\sin(x^{k3}) + \sin((x \cdot k4)^{k5}) + x \cdot k6},$$

- therein mean:

- x = computational substitute for time t during a main pulse;
- k1 = offset value;
- k2 = amplitude factor, k2≠0;
- k3 = exponent of x, k3≠0;
- k4 = multiplication factor of x, k4≠0;
- k5 = exponent of (x*k4), k5≠0;
- k6 = multiplication factor of x;
- wherein k1 - k6 are parameters which are freely selectable within defined limits to give different shapes to the amplitude waveform, each main pulse being modulated with sub-pulses (11) by respective selection of the parameters.

2. The device according to claim 1, the stress pattern is formed by a temporally defined occurrence of the signal events.

3. The device according to claim 1 or 2, wherein at least one group of similar signal events from the sequence of signal events occurs periodically.

4. The device according to one of the preceding claims, wherein the stress pattern defines a clock measure, in particular by selected signal events from the sequence of signal events.

5. The device according to the preceding claim, wherein a tempo of the clock measure varies, in particular increases or decreases.

6. The device according to one of the preceding claims, wherein the stress pattern in the rhythmic signal (15) repeats at least 10 times per minute, in particular at least 50 times per minute .

7. The device according to one of the preceding claims, wherein the signal sequence comprises a length between 45 s and 20 min, in particular between 90 s and 25 min.

8. The device according to one of the preceding claims, wherein the signal events comprise a frequency range such that a reproduction of the rhythmic signal via an electroacoustic transducer results in an audible sound and/or an infrasound.

9. The device according to any of the preceding claims, wherein the signal events are formed by at least one of the following:

   - a breathing sound;
   - a blood flow sound;
   - a sound of a heartbeat;
   - a sound of a percussion instrument;
   - a sound of a stringed instrument;
   - a sound of a wind instrument.

10. The device according to one of the preceding claims, wherein the computational substitute x depends linearly on the time t.

11. The device according to one of the preceding claims, wherein each main pulse (10a, 10b) comprises a pulse length, and the computational substitute x for the time t is defined as

$$x = x1 + \frac{x2 - x1}{T_i} \cdot (t - t0_i),$$

with

x1 = initial value for x;
x2 = final value for x;
Ti = pulse length of a main pulse; and
t0i = start time of the main pulse.

12. The device according to one of the preceding claims, wherein

   - the signal sequence (13) is formed from a superposition of a first sequence (13a) of main pulses (10a), at least one second sequence (13b) of main pulses (10b) and the rhythmic signal;
   - the amplitude waveform of the main pulses comprises the function y(x);
   - each main pulse (10a, 10b) comprises a pulse length;
   - the pulse length and the pulse repetition rate of the main pulses (10b) of the second sequence (13b) of main pulses (10b) are different from the pulse length and the pulse repetition rate of the main pulses (10a) of the first sequence (13a) of main pulses (10a)
   - the pulse length of the main pulses (10b) of the second sequence (13b) of main pulses (10b) changes monotonically in a time-dependent course, in particular decreases monotonically or increases monotonically; and
- the pulse repetition rate of the main pulses (10b) of the second sequence (13b) of main pulses (10b) changes monotonically in a time-dependent course opposite to the change in the pulse length, in particular increases monotonically or decreases monotonically.

13. The device according to the preceding claim, wherein

- the main pulses (10a) of the first sequence (13a) of main pulses (10a) follow one another directly in the time-dependent course, in particular without a pause; and
- the main pulses (10b) of the second sequence (13b) of main pulses (10b) follow one another directly in the time-dependent course, in particular without a pause.

14. The device according to one of claims 12 or 13, wherein

- the pulse length of the main pulses (10a) of the first sequence of main pulses (10a) changes monotonically in the time-dependent course, in particular decreases monotonically or increases monotonically; and
- the pulse repetition rate of the main pulses (10a) of the first sequence of main pulses (10a) changes monotonically in the time-dependent course opposite to the change in the pulse length of these main pulses (10a), in particular increases monotonically or decreases monotonically.

## Patentansprüche

1. Vorrichtung zur Beeinflussung biologischer Abläufe in einem lebenden Gewebe, insbesondere einem menschlichen Körper, zur Beaufschlagung zumindest eines Teils des Gewebes mit einem pulsierenden magnetischem Feld, mit einer Felderzeugungsvorrichtung (2) zur Erzeugung des pulsierenden magnetischen Feldes und einem Impulsgenerator (1) zur Ansteuerung der Felderzeugungsvorrichtung (2) mit einer Signalfolge (13), wobei der Impulsgenerator (1) zur Abgabe der Signalfolge (13) ausgebildet ist, welche gebildet ist aus einer Überlagerung wenigstens einer Folge (13a) von Hauptimpulsen (10a), deren Impulswiederholungsrate zwischen 0,1 und 1000 Hz liegt, insbesondere zwischen 3 und 300 Hz liegt, und eines Rhythmiksignals,
wobei

- das Rhythmiksignal eine Folge von Signalereignissen aufweist, die in wenigstens einem regelmäßigen Betonungsmuster organisiert sind; und
- der Amplitudenverlauf eines Hauptimpulses die folgende Funktion y(x) aufweist:

$$y(x) = k1 + k2 \cdot e^{\sin(x^{k3}) + \sin((x \cdot k4)^{k5}) + x \cdot k6};$$

- darin bedeuten:

  - x = rechnerische Ersatzgröße für die Zeit t während eines Hauptimpulses;
  - k1 = Offsetwert;
  - k2 = Amplitudenfaktor, k2≠0;
  - k3 = Exponent von x, k3≠0;
  - k4 = Multiplikationsfaktor von x, k4≠0;
  - k5 = Exponent von (x*k4), k5≠0;
  - k6 = Multiplikationsfaktor von x;
  - wobei k1 - k6 Parameter sind, die in festgelegten Grenzen frei wählbar sind, um dem Amplitudenverlauf unterschiedliche Formen zu geben, wobei jeder Hauptimpuls durch entsprechende Wahl der Parameter mit Unterimpulsen (11) moduliert ist.

2. Vorrichtung nach Anspruch 1, wobei das Betonungsmuster durch ein zeitlich definiertes Auftreten der Signalereignisse gebildet ist.

3. Vorrichtung nach Anspruch 1 oder 2, wobei wenigstens eine Gruppe von gleichartigen Signalereignissen aus der Folge von Signalereignissen periodisch auftritt.

4. Vorrichtung nach einem der vorhergehenden Ansprüche, wobei das Betonungsmuster ein Taktmaß definiert, insbesondere durch ausgewählte Signalereignisse aus der Folge von Signalereignissen.

5. Vorrichtung nach dem vorhergehenden Anspruch, wobei ein Tempo des Taktmaßes variiert, insbesondere ansteigt oder abfällt.

6. Vorrichtung nach einem der vorhergehenden Ansprüche, wobei sich das Betonungsmuster im Rhythmiksignal (15) wenigstens 10 mal pro Minute wiederholt, insbesondere wenigstens 50 mal pro Minute.

7. Vorrichtung nach einem der vorhergehenden Ansprüche, wobei die Signalfolge eine Länge zwischen 45 s und 20 min aufweist, insbesondere zwischen 90 s und 25 min.

8. Vorrichtung nach einem der vorhergehenden Ansprüche, wobei die Signalereignisse einen Frequenzbereich aufweisen derart, dass bei einer Wiedergabe des Rhythmiksignals über einen elektroakustischen Umsetzer ein Hörschall und/oder ein Infraschall resultiert.

9. Vorrichtung nach einem der vorhergehenden Ansprüche, wobei die Signalereignisse gebildet sind durch wenigstens eines der folgenden:

- ein Atemgeräusch;
- ein Blutströmungsgeräusch;
- ein Geräusch eines Herzschlags;
- ein Geräusch eines Schlaginstruments;
- ein Geräusch eines Saiteninstruments;
- ein Geräusch eines Blasinstruments.

10. Vorrichtung nach einem der vorhergehenden Ansprüche, wobei die rechnerische Ersatzgröße x linear von der Zeit t abhängt.

11. Vorrichtung nach einem der vorhergehenden Ansprüche, wobei jeder Hauptimpuls (10a, 10b) eine Impulslänge aufweist, und die rechnerische Ersatzgröße x für die Zeit t definiert ist als

$$x = x1 + \frac{x2 - x1}{T_i} \cdot (t - t0_i),$$

mit

x1 = Anfangswert für x;
x2 = Endwert für x;
Ti = Impulslänge eines Hauptimpulses; und
t0i = Startzeit des Hauptimpulses.

12. Vorrichtung nach einem der vorhergehenden Ansprüche, wobei

- die Signalfolge (13) gebildet ist aus einer Überlagerung einer ersten Folge (13a) von Hauptimpulsen (10a), wenigstens einer zweiten Folge (13b) von Hauptimpulsen (10b) und des Rhythmiksignals;
- der Amplitudenverlauf der Hauptimpulse die Funktion y(x) aufweist;
- jeder Hauptimpuls (10a, 10b) eine Impulslänge aufweist;
- die Impulslänge und die Impulswiederholungsrate der Hauptimpulse (10b) der zweiten Folge (13b) von Hauptimpulsen (10b) sich unterscheiden von der Impulslänge und der Impulswiederholungsrate der Hauptimpulse (10a) der ersten Folge (13a) von Hauptimpulsen (10a);
- die Impulslänge der Hauptimpulse (10b) der zweiten Folge (13b) von Hauptimpulsen (10b) sich im zeitlichen Verlauf monoton verändert, insbesondere monoton abfällt oder monoton ansteigt; und
- die Impulswiederholungsrate der Hauptimpulse (10b) der zweiten Folge (13b) von Hauptimpulsen (10b) sich im zeitlichen Verlauf entgegengesetzt zur Veränderung der Impulslänge monoton verändert, insbesondere monoton ansteigt oder monoton abfällt.

13. Vorrichtung nach dem vorhergehenden Anspruch, wobei

- die Hauptimpulse (10a) der ersten Folge (13a) von Hauptimpulsen (10a) im zeitlichen Verlauf unmittelbar aufeinanderfolgen, insbesondere ohne Pause; und
- die Hauptimpulse (10b) der zweiten Folge (13b) von Hauptimpulsen (10b) im zeitlichen Verlauf unmittelbar aufeinanderfolgen, insbesondere ohne Pause.

14. Vorrichtung nach einem der Ansprüche 12 oder 13, wobei

- die Impulslänge der Hauptimpulse (10a) der ersten Folge von Hauptimpulsen (10a) sich im zeitlichen Verlauf monoton verändert, insbesondere monoton abfällt oder monoton ansteigt; und
- die Impulswiederholungsrate der Hauptimpulse (10a) der ersten Folge von Hauptimpulsen (10a) sich im zeitlichen Verlauf entgegengesetzt zur Veränderung der Impulslänge dieser Hauptimpulse (10a) monoton verändert, insbesondere monoton ansteigt oder monoton abfällt.

## Revendications

1. Dispositif destiné à influencer des processus biologiques dans un tissu vivant, en particulier dans le corps humain, pour appliquer un champ magnétique pulsé à au moins une partie du tissu, comprenant un dispositif de génération de champ (2) destiné à générer le champ magnétique pulsé et un générateur d'impulsions (1) destiné à commander le dispositif de génération de champ (2) à l'aide d'une séquence de signaux (13), le générateur d'impulsions (1) étant adapté pour délivrer la séquence de signaux (13), qui est formée d'une superposition d'au moins une séquence (13a) d'impulsions principales (10a), dont la fréquence de répétition des impulsions est comprise entre 0,1 et 1 000 Hz, en particulier entre 3 et 300 Hz, et d'un signal rythmique,
   dans lequel :

   - le signal rythmique comprend une séquence d'événements de signal organisés selon au moins un motif d'accentuation régulier ; et
   - une forme d'onde d'amplitude d'une impulsion principale comprend la fonction suivante y(x) :

   $$y(x) = k1 + k2 \cdot e^{\sin(x^{k3}) + \sin((x \cdot k4)^{k5}) + x \cdot k6};$$

   - où :

      - x = substitut de calcul pour le temps t pendant une impulsion principale ;
      - k1 = valeur de décalage ;
      - k2 = facteur d'amplitude, k2 ≠ 0 ;
      - k3 = exposant de x, k3 ≠ 0 ;
      - k4 = facteur de multiplication de x, k4 ≠ 0 ;
      - k5 = exposant de (x*k4), k5 ≠ 0 ;
      - k6 = facteur de multiplication de x ;
      - où k1 à k6 sont des paramètres librement sélectionnables dans des limites définies pour donner différentes formes à la courbe d'amplitude, chaque impulsion principale étant modulée par des sous-impulsions (11) par la sélection respective des paramètres.

2. Dispositif selon la revendication 1, dans lequel le motif d'accentuation est formé par une occurrence définie dans le temps des événements de signal.

3. Dispositif selon la revendication 1 ou 2, dans lequel au moins un groupe d'événements de signal similaires issus de la séquence d'événements de signal se produit périodiquement.

4. Dispositif selon l'une des revendications précédentes, dans lequel le motif d'accentuation définit une mesure d'horloge, en particulier par des événements de signal sélectionnés parmi la séquence d'événements de signal.

5. Dispositif selon la revendication précédente, dans lequel le tempo de la mesure d'horloge varie, en particulier augmente ou diminue.

**6.** Dispositif selon l'une des revendications précédentes, dans lequel le motif d'accentuation dans le signal rythmique (15) se répète au moins 10 fois par minute, en particulier au moins 50 fois par minute.

**7.** Dispositif selon l'une des revendications précédentes, dans lequel la séquence de signaux a une durée comprise entre 45 s et 20 min, en particulier entre 90 s et 25 min.

**8.** Dispositif selon l'une des revendications précédentes, dans lequel les événements de signal comprennent une gamme de fréquences telle qu'une reproduction du signal rythmique via un transducteur électroacoustique donne lieu à un son audible et/ou à un infrason.

**9.** Dispositif selon l'une des revendications précédentes, dans lequel les événements de signal sont formés par au moins l'un des éléments suivants :

- un son de respiration ;
- un son de circulation sanguine ;
- un son de battement cardiaque ;
- un son d'instrument à percussion ;
- un son d'instrument à cordes ;
- un son d'instrument à vent.

**10.** Dispositif selon l'une des revendications précédentes, dans lequel le substitut de calcul x dépend linéairement du temps t.

**11.** Dispositif selon l'une des revendications précédentes, dans lequel chaque impulsion principale (10a, 10b) comprend une longueur d'impulsion, et le substitut de calcul x pour le temps t est défini comme suit :

$$x = x1 + \frac{x2 - x1}{T_i} \cdot (t - t0_i),$$

avec

x = x1 + (x2 - x1) / T_i · (t - $t_0$_i), avec
x1 = valeur initiale de x ;
x2 = valeur finale de x ;
Ti = durée d'une impulsion principale ; et
t0i = instant de début de l'impulsion principale.

**12.** Dispositif selon l'une des revendications précédentes, dans lequel

- la séquence de signaux (13) est formée par la superposition d'une première séquence (13a) d'impulsions principales (10a), d'au moins une deuxième séquence (13b) d'impulsions principales (10b) et du signal rythmique ;
- la forme d'onde d'amplitude des impulsions principales comprend la fonction y(x) ;
- chaque impulsion principale (10a, 10b) comprend une durée d'impulsion ;
- la durée d'impulsion et la fréquence de répétition des impulsions principales (10b) de la deuxième séquence (13b) d'impulsions principales (10b) sont différentes de la durée d'impulsion et de la fréquence de répétition des impulsions principales (10a) de la première séquence (13a) d'impulsions (10a) ;
- la durée des impulsions principales (10b) de la deuxième séquence (13b) d'impulsions principales (10b) varie de manière monotone selon une courbe dépendante du temps, en particulier diminue de manière monotone ou augmente de manière monotone ; et
- la fréquence de répétition des impulsions principales (10b) de la deuxième séquence (13b) d'impulsions principales (10b) varie de manière monotone selon une courbe dépendante du temps, dans le sens opposé à la variation de la durée d'impulsion, en particulier augmente de manière monotone ou diminue de manière monotone.

**13.** Dispositif selon la revendication précédente, dans lequel

- les impulsions principales (10a) de la première séquence (13a) d'impulsions principales (10a) se succèdent directement dans le déroulement dépendant du temps, en particulier sans pause ; et
- les impulsions principales (10b) de la deuxième séquence (13b) d'impulsions principales (10b) se succèdent directement dans le déroulement dépendant du temps, en particulier sans pause.

14. Dispositif selon l'une des revendications 12 ou 13, dans lequel :

- la durée des impulsions principales (10a) de la première séquence d'impulsions principales (10a) varie de manière monotone au cours du temps, en particulier diminue de manière monotone ou augmente de manière monotone ; et
- la fréquence de répétition des impulsions principales (10a) de la première séquence d'impulsions principales (10a) varie de manière monotone au cours du temps, dans le sens opposé à la variation de la durée de ces impulsions principales (10a), en particulier augmente de manière monotone ou diminue de manière monotone.

# Fig 1

Fig 2

# Fig 3

# Fig 4

10a

x1
t0a

t

x2
t0a + Ta

10b

x1
t0b

t

x2
t0b + Tb

# Fig 5

# Fig 6

13c

t

15

t

13

t

Fig 7

**EP 4 284 499 B1**

**Patent documents cited in the description**

- EP 2050481 B1
- EP 0594655 B1
- EP 0729318 B1
- EP 0995463 B1